# EUROPEAN PATENT APPLICATION

(11) **EP 2 634 715 A1**
(43) Date of publication of application: **04.09.2013**
(21) Application number: 12157801.7
(22) Date of filing: 01.03.2012
(51) Int. Cl.: G06F 19/00

(54) **Computer-implemented method and system for generating a report**

(71) Applicant: Agfa HealthCare, 2640 Mortsel (BE)
(72) Inventor: Felix, Joost, 2640 Mortsel (BE); Kieckens, Wannes, B-2640 Mortsel (BE)

(57) **Abstract**

A report generating method and system with different modes to complete a report template such as insertion of a transcribed audio file originating from digital dictation, data base retrieval, text input, input of measurement results.

## Description

### FIELD OF THE INVENTION

The present invention relates to a method and a system for generating a report such as a medical report.

### BACKGROUND OF THE INVENTION

In the field of healthcare it is common practice to generate medical reports including medical findings and examination data. Medical written reports are commonly generated by dictation of the content of the report by the physician followed by either by transcription of the dictation by a transcriptionist or followed by conversion of the dictation into text by means of a speech recognition system.

When doctors, e.g. radiologists make reports using digital dictation in particular, they usually have some kind of report structure in mind with sections such as Procedure, Findings, Conclusion, Recommendations etc.

The transcriptionist makes these report structures explicit when he/she transcribes the dictation. When report structures have been configured for specific contexts the transcriptionist is presented with them during transcription. In that case it is important that the report structure matches the structure that the radiologist had in mind while dictating, however the radiologist does not see the report structure. It is thus very well possible that the structure the radiologist has in mind does not match with the structure the transcriptionist is trying to reflect in the report.

In case of dictation followed by speech recognition, the radiologist can see the transcribed text immediately appear in the sections of the report. In this case there is no problem.

Also in the case where the report is generated by plain text editing, there is no such problem.

Examples of state of the art methods for the generation of medical reports using digital dictation are described below.

### EXAMPLE 1:

Peter Lambert, radiologist, dictates a report for a CT Chest by means of digital dictation.
Peter always works with digital dictation.
The report contains abnormal findings.
The active report will be a variation on the report of the latest comparison study, only the measurements will be different.
Miranda, the transcriptionist transcribes it.

The conventional way of working with digital dictation:
Peter has one report structure defined for all his reports, with one section. In the report pane he sees the dictation controls: the progress bar and the following buttons: record/stop recording, replay, back to front, rewind, fast forward, to end,
insert/overwrite. He can change the method of dictation.

Even though the active report structure only contains one section, for CT Chest Peter has a structure in mind with 5 sections: Reason for study, Procedure details, Findings, Recommendations, Conclusion. He dictates the section titles as he goes along, hoping that the transcriptionist will recognize them as section titles.
He would very much like to copy the content of the latest comparison study to the active report but he cannot do this during digital dictation. By changing to text only he can overcome this limitation. However, that would mean that he can no longer switch back to digital dictation, because in that case all typed text would be lost.

So Peter starts dictating the reason for study, the procedure details, and other data that could have been retrieved from the database with an autofill feature.
He dictates the measurements he has made in image area as he cannot import these data.

When he is ready he sends the dictation to the Miranda. She finds the transcription task "CT Chest" in her To Do list. When she starts it she gets the dictation controls in one pane and a report structure with one section in another pane. Above the section is the report toolbar with formatting features, text macros, report structures and more.
The printing template with different sections that is used by the radiologists using speech recognition cannot be used so Miranda has to apply the formatting of the sections herself as her report only contains one section. Alternatively she can switch to a report structure with the sections that the radiologist dictates, but she only finds out which sections have been used after listening to the complete dictation.

If she has the permissions to do so she can sign off. In many countries however it is required by law that she sends the report back to the radiologist to sign off for acceptance and release. This slows down the reporting time.

### Example 2:

Peter Lambert, radiologist, dictates a report for a CT Chest by means of digital dictation.
Miranda, the transcriptionist will transcribe it.
Peter Lambert always works with digital dictation.
The report comprises "Normal" findings.

The conventional way of working with digital dictation:
For "normal" findings, Peter Lambert would like to use the "normal report" feature and sign off. He cannot use the normal reports as he uses digital dictation with one report section. If he has defined normal reports with one section he can tell Miranda to insert a "normal" report. However, he might still want to change some details. In that case he needs to wait until the report comes back to him as a Sign off task.
Most likely he will dictate the whole report, all 5 sections he has in mind. This means that he also has to dictate the input for those report sections that radiologists who would use online speech recognition can get pre-filled.
If Miranda has the permissions to sign off he can also tell her to go ahead and sign off.
Peter wants to see for himself if the report was transcribed correctly, gets a sign off task, reads the report and signs off.

From these two examples can be concluded that the digital dictation mode, when e.g. compared to speech recognition or plain text editing, is less convenient because the radiologist cannot see the report structures he has in mind or that have been pre-designed.

Furthermore, he does not have the possibility of inserting data that have already been filled in in previous reports or that are available in the system and would have been pre-filled when using speech recognition. No copying, inserting, changing functionality is available.

Still furthermore, the fact that digital dictation requires the intermediary of a transcriptionist has as a consequence that the radiologist cannot sign off the report immediately after generating the report data. This work flow may delay the process of report generation.

All the above drawbacks make the prior art workflow for the radiologist cumbersome, slow, inconvenient and error prone.

It is an aspect of the present invention to provide a method and a system that overcome the drawbacks of the prior art methods and systems.

### SUMMARY OF THE INVENTION

The above-mentioned aspects are realised by a method and a system as set out in the appending independent claims.

Specific features for preferred embodiments of the invention are set out in the dependent claims.

The method of the present invention relates to a method and system for generating a report, such as a medical report.

According to the present invention, when a user dictates a report he gets to see the dictation controls and additionally disposes of the tools for typing data and inserting data. The latter are a report toolbar, a report structure (with report sections), a text macro pane etc.

The invention is advantageous in that the user who provides the input for the report may have the same view as the transcriptionist. Furthermore, the user the digital dictation mode has additional functionality for report generation compared to prior art digital dictation work flow.
The user has the opportunity to sign off the report so that the time between start of report generation and signing off is reduced.

Still furthermore, repetitive work can be avoided.

The above examples of situations encountered when a radiologist is generating a report using digital dictation are solved according to the present invention in the following way.

### Example 1:

Peter can now use in addition to digital dictation the same report structures that the radiologists using online speech recognition use. For the current context - CT Chest, reading, radiologists - he sees 5 report sections on the screen: Reason for study (pre-filled), Procedure details (pre-filled), Findings, Recommendations and Conclusion. He can copy the content of the report of the latest comparison study to the active report. He can insert new measurements he makes on a displayed image into the active report section. After changing some small details he can sign off (indicate that he approves the content of the report) immediately, possibly skipping transcription.

### Example 2:

Peter can now use the same report structure for CT Chest that the radiologists using online speech recognition use which is in this case a structure with 5 sections. He can insert measurements linked to the acquired and displayed image, copy content from a comparison study, insert text macros, possibly structured fill-in forms, skip dictating the reason for study, the procedure details and other pre-filled texts and he can get a print preview. When he finds his dictation is ready for sign off he can skip sending the report to the transcriptionist and sign off immediately.

The method of the present invention is generally implemented in the form of a computer program product adapted to carry out the method steps of the present invention when run on a signal processing means such as a computer. The computer program product is commonly stored in a computer readable carrier medium such as a DVD. Alternatively the computer program product takes the form of an electric signal and can be communicated to a user through electronic communication.

While the subject matter of the present invention has been developed for the generation of medical reports, it will be clear that it can be used for generation of all types documents for which digital dictation is used. Examples of such documents are medical and non-medical reports of all kinds, legal documents, etc.

Further advantages and embodiments of the present invention will become apparent from the following description and drawings.

### BRIEF DESCRIPTION OF THE DRAWINGS

Fig. 1 shows a screen shot of a display screen according to the prior art on which dictation controls for the digital dictation mode are displayed,
Fig. 2 is a screen shot illustrating the report template and the functionalities the user disposes of when the present invention is applied,
Fig. 3, 4 and 5 show further functionalities of the present invention,
Fig. 6 shows inputs to a report generating application.

### DETAILED DESCRIPTION OF THE INVENTION

While the present invention will hereinafter be described in connection with preferred embodiments thereof, it will be understood that it is not intended to limit the invention to those embodiments.

According to the present invention a system and method is disclosed for generating a report such as a medical report. In the following description reference is made to a medical report generated by a radiologist. It is however clear that the invention is not meant to be limited to a medical report, nor to a radiologist generating it. The general aspects of the present invention are also applicable to the generation of other types of reports and to other kinds of users.

A system according to the present invention commonly comprises a workstation with a display screen for displaying the report. A report generating application is run on a computer coupled to the workstation. The computer is arranged to have access to a report template repository as well as to databases comprising data which might be filled in in the report and to other applications run on the computer. The system is typically part of a picture archiving and communication system (PACS) such as Agfa IMPAX or of a PACS system with an integrated Radiology information system.

When the report generating application is run, a report template is retrieved from a template repository and displayed on the display screen of the workstation.

In the context of the present invention, a template defines a report structure (e.g. how many paragraphs will be in the final report) and lay-out (bulleted lists etc.). It may also contain content such as text, dynamic context information (data field) etc.

The template is preferably pre-configured for a user (such as for a specific doctor) or an examination type (such as CT head, CT abdomen) (or occasionally for a patient) or a combination thereof.

The template has fields to be filled in with new information concerning an actual examination but has also fields which are filled in automatically such as fields regarding the identity of the patient. This information is commonly provided by a radiology information system (RIS) or a hospital information system (HIS).

Templates in the context of the present invention may also consist of an earlier report in which certain fields are already filled in.

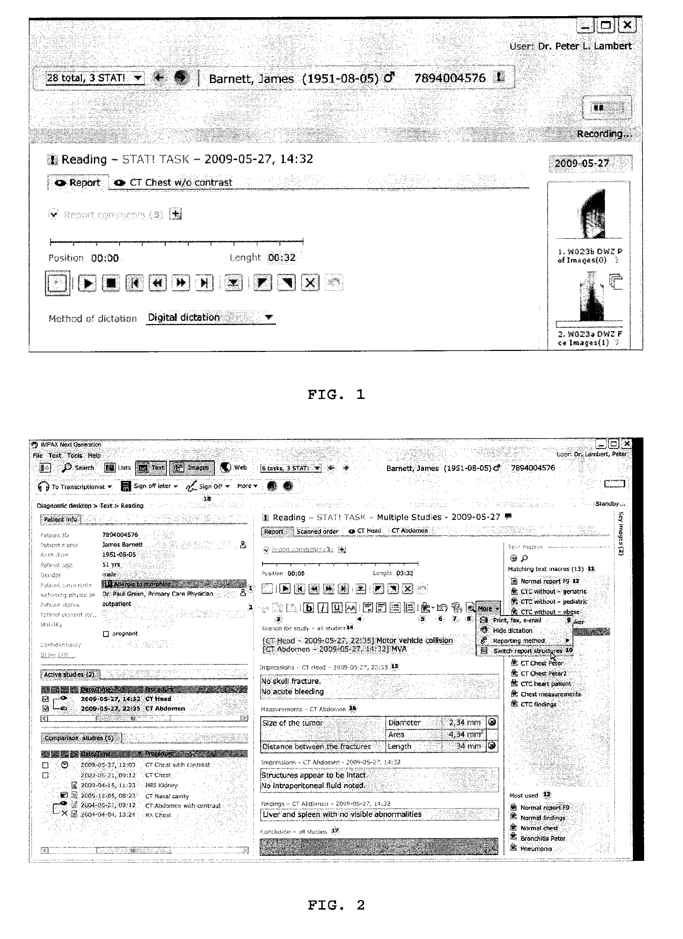

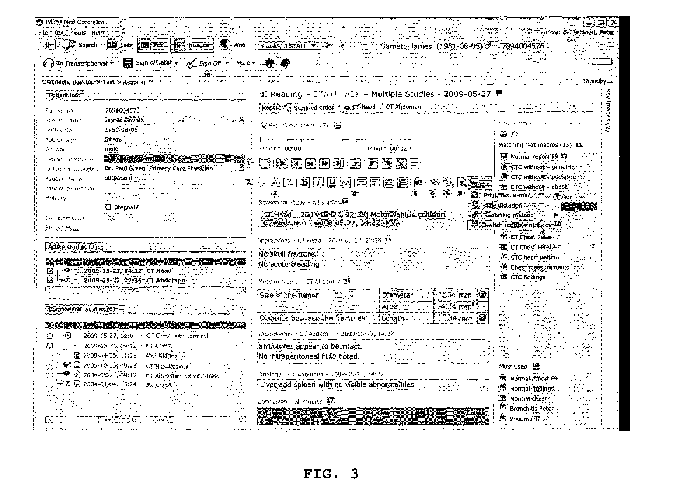

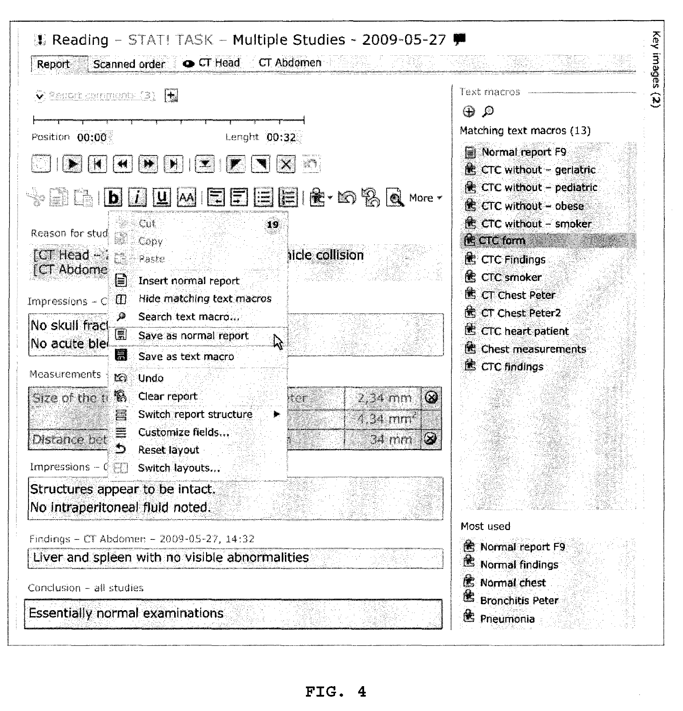

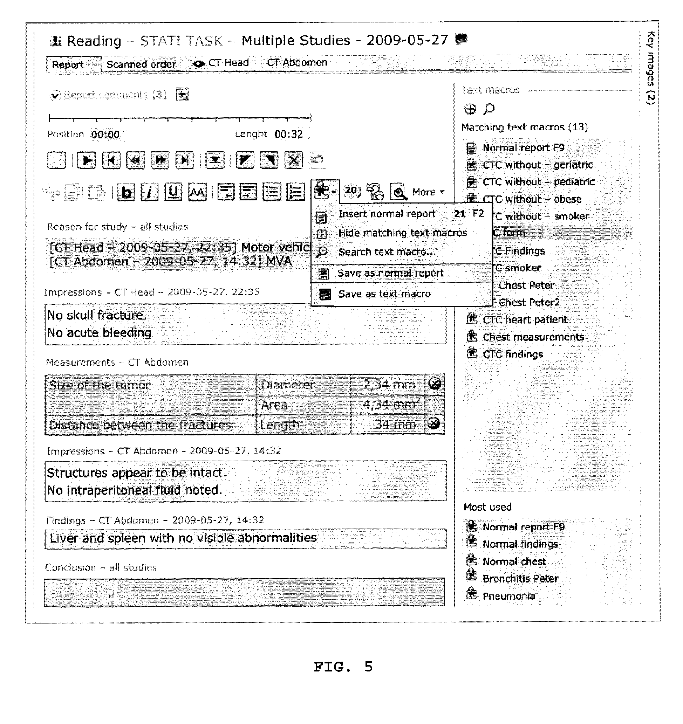

An example of a template is shown in figures 2 - 5.

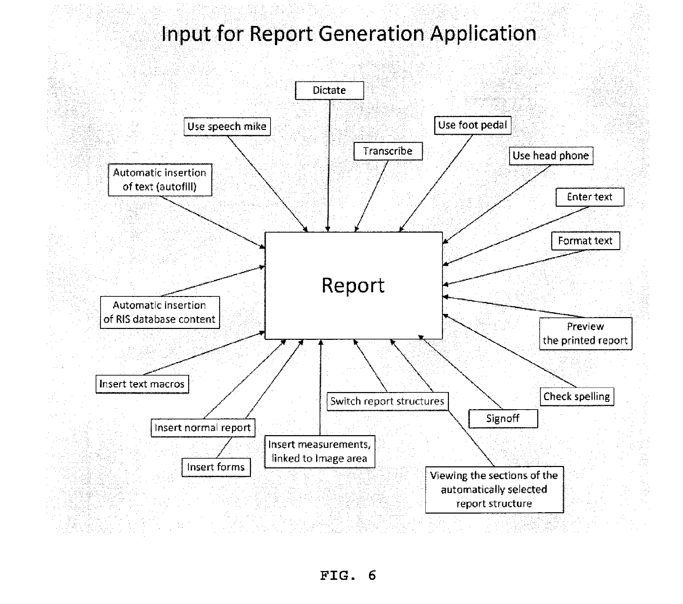

The report generating application may receive input from a number of devices such as illustrated in figure 6.

One of the data input device applicable in the context of the present invention is a device for digital dictation comprising a speech microphone and dictation software. Digital dictation systems are known in the art. Digital dictation data input can for example be implemented based on the Java Sound API.

Digital dictation control is integrated in a text area on the display screen of the workstation. The digital dictation control consists of buttons to record and playback audio. In the described embodiment additional information is displayed concerning the current position and the total length of a recorded audio fragment. This information is made visible in the form of a displayed slider. Alternatively it can be made visible in the form of text. Navigating back and forth in an audio fragment can be done via winding buttons (on screen or on a speech microphone) or by moving the slider.
Recording can be controlled not only by the control buttons on the screen but also via buttons on a speech microphone or on a foot pedal or other dedicated hardware means.

Actual recording can be performed either online or off-line. In an off line recording situation a doctor may use a dictaphone or smart phone or the like to make dictations which are then imported and/or combined with the other aspects described below.

An audio file generated by digital dictation is converted into text which may then be added to the relevant fields of the template.
For this purpose the audio file may be sent to a transcriptionist (e.g the doctor's secretary).
The transcriptionist preferably disposes of the same template as used by the doctor. By providing the same template to the doctor and to the transcriptionist the workflow is optimized, errors are avoided and the time required to finish the report can be decreased.

In a specific embodiment it is possible to provide a separate audio file for each of a number of data fields.

Other data input means are placed at the doctor's disposal when using the report generation method of the present invention.

Data repository devices (databases) coupled to the report generating application may be a source of data input to the application.

Still another mode of operation for data entry in the template is text entry. For this purpose preferably common text processing techniques are available. Macro's can also be used.

In one embodiment of a report generating method and system according to the present invention relating to medical reports, the report generating application is also coupled to an application running on the computer for displaying an image obtained by image acquisition techniques such as digital radiography, computed radiography, CT imaging etc. and to an application for performing measurements on a displayed image. The image and measurement tools and results are displayed on a different display pane referred to as image area or image pane.

In the present invention data for inserting into the above-described template may be generated by measurements performed in the image pane. The report generating application and the applications running in the image pane are arranged to allow data transfer between them. Measurement results may thus be copied into dedicated sections of the report template. In this way re-typing is avoided and errors are thus avoided.

Upon completion of a report template by adding data, the completed report may be signed off by the radiologist (for completion and acceptance). The report can then be archived, transmitted or printed.

An example of individual elements which can be provided are shown in figures 2 - 5, there function or goal is numbered and have the following meaning:
1. dictation controls: record, playback
2. report toolbar
3. cut, copy, paste
4. Text formatting tools: bold, italics, underline, to uppercase, indent, outdent, bullets, numbering
5. Text macros menu
6. Undo last action
7. Clear report
8. Preview
9. More - print/fax/e-mail
10. More - Switch report structures
11. Matching text macros
12. Normal report
13. Most used text macros
14. Pre filled section, not editable
15. Editable section
16. Measurement section, linked to Image area and only editable from there
17. Required section
18. sign off button
19. shortcut menu on a report section

During report creation reporting according to this invention offers the following benefits:
The report structures can vary with the context:
   Example 1: A mammo report and a report on an RX Thorax may contain some very different sections.
   Example 2: Dr. Peter Lambert may use a different report structure for CT than Dr. Amy Green.
If the report structure (template) that was configured for the context doesn't fulfill the radiologist's needs he can replace it by another template (10).

The radiologist can use a normal report (12) that nicely fills in all report sections in the active report structure and can sign off immediately without transcription or correction by the secretary. Or he can use a normal report and make some small changes to it and then sign off.

He can use text macros (5) that are linked to the active report structure or to the active report section. These can be fill-in forms.

Some report sections can be pre-filled with database information (reason for study, procedure details,...) (14).

He can transfer measurements from Image area to an ad hoc report section that shows measurements (16).

Some sections may be mandatory, such as Conclusion. The radiologist gets a visual indication of this item (17).

When viewing a comparison report, the reporting method of the present invention makes it is possible to view the Conclusion section only.

Through the report toolbar a range of other features are available: cut, copy and paste (3), formatting (4), undo (6), clear report (7), voice commands and keyboard shortcuts, preview (8), print/fax/e-mail (9).

## Claims

1. A computer-implemented method for generating a report comprising the steps of
- selecting a digital dictation mode thereby displaying on a display screen operation control buttons arranged for controlling digital dictation,
- retrieving a template for said report with a number of configured data fields from a template repository and displaying said template on said display screen,
- filling in data fields in said template by means of data generated by performing one or more of following actions:
- retrieving data from a data base,
- generating at least one audio file by means of digital dictation and transcribing said audio file(s)into text,
- generating said data as text by means of text processing techniques.

2. A method according to claim 1 wherein said report is signed off.

3. A method according to claim 1 wherein a separate audio file is generated for each of a number of said data fields.

4. A method according to claim 1 wherein said report is a medical report and said data are retrieved from a hospital or radiology information system.

5. A method according to claim 1 wherein said report is a medical report and said data are results of measurements performed on a displayed image generated by a medical examination.

6. A system for generating a report comprising
- a workstation with a display screen coupled to a computer,
- means for performing digital dictation coupled to said workstation,
- a data repository comprising a number of templates for said report, said templates comprising a number of pre-configured data fields,
- means for selecting a template from said repository and displaying it on said screen,
- means for entering data into said pre-configured data fields, said data being generated by at least one of the following actions:
- retrieval from a data base,
- text entering by means of text processing techniques,
- transcribing said audio file.

7. A system according to claim 6 comprising means for signing off a generated report.

8. A system according to claim 6 comprising means for retrieving measurement results from an application for performing measurements on an image.

9. A system according to claim 6 wherein said report is a medical report.

10. A system according to claim 6 wherein said report is a medical report and said image is a medical image.

11. A computer program product adapted to carry out the method of any of claims 1 - 5 when run on a computer.

12. A computer readable medium comprising computer executable program code adapted to carry out the steps of any of claims 1 - 5.
